# EUROPEAN PATENT APPLICATION

(11) **EP 1 046 390 A1**
(43) Date of publication of application: **25.10.2000**
(21) Application number: 00303314.9
(22) Date of filing: 19.04.2000
(51) Int. Cl.: A61K 7/06

(54) **Compositions and methods for cleaning and removing contaminants from hair**

(30) Priority: 20.04.1999 US 294558
(71) Applicant: Calgon Corporation, Naperville, Illinois 60563-1198 (US)
(72) Inventor: Matz, Gary F., Carnegie, PA 15106 (US)
(74) Representative: Baillie, Iain Cameron

(57) **Abstract**

A composition for use in removal of multivalent metals, minerals, environmental dirt, and sebum from hair is disclosed herein which comprises the combination of an acidic pH and a dispersant in water and, optionally, an inorganic reducing agent, a chelating agent, a gelling agent, and surfactants. Also disclosed herein is a method for removal of mineral residues from hair by use of compositions as disclosed.

## Description

The present invention relates to a composition and method of using the composition for treating and/or cleansing human hair of mineral deposits, environmental dirt, sebum, and multivalent metal ions such as copper, iron, lead, zinc, aluminum, cadmium, and those from water hardness minerals such as calcium, magnesium and others.

Deposition of minerals in hair is an unavoidable phenomenon. Well water and surface water, used by many communities around the country and around the world, is enriched with a wide array of minerals. Copper and iron ions can also leach from pipes into water, especially where water supplies are chlorinated periodically. Swimmers encounter a constant source of copper in the form of certain algicides added frequently to swimming pools.

Hair is composed of keratin, a sulfur-containing fibrous protein. The isoelectric point of keratin, and more specifically of hair, is generally in the pH range of 3.2-4.0. Therefore, at the pH that hair is typically exposed to during bathing, swimming and other activities (about 5.5-7.5), hair carries a net negative charge. Consequently, multivalent metal ions due to their positive charge are attracted to the negatively charged surface of the hair.

Hair is a strong adsorbent of these metals. Binding is so strong that once these multivalent cations are captured by the anionic sites on the fiber, they are hard to elute from these sites. As a result, there is a gradual metallic build-up, which only intensifies with time. The extent to which minerals in general bind to hair depends on several factors such as condition and porosity, length of the fiber, and levels as well as duration of exposure.

Common mineral residues such as iron, copper and lead produce obvious discoloration of hair, in which iron is reddish orange, copper is green and lead is black. Calcium and magnesium are not colored, but manifest their presence by giving the hair a dry, brittle feel. These minerals form deposits that can not be removed from the hair and skin through normal processes and techniques.

This metal build-up can lead to a range of undesirable, and sometimes adverse, effects. Metals like copper, lead and iron, can interfere with chemical treatments such as hair coloring and permanent waving. Iron- and/or copper-contaminated hair may experience uneven and unpredicted color deposit because of variations in hair porosity and mineral build-up. Such variations in mineral build-up lead to differences in the rate of catalysis and formation of dye molecules along the length of the hair shaft, causing the uneven color deposit. Iron can also interfere with permanent waving, by reacting with reducing agents such as thioglycolic acid and its derivatives, to produce an undesirable purple color. Both iron and copper can catalyze the decomposition of the peroxide in the neutralizing solution of the rebonding step, hence reducing its capacity to fully reoxidize the thiol groups. This can lead to a weakened perm and a fragile hair.

U.S. Pat. No. 4,581,229, teaches the use of a composition based on lanthanum salts for removing heavy metals from hair. This reference, however, only discloses the removal of very low levels (less than 200ppm) of ferric oxide from the hair. The patent also warns against the use of chelating and complexing agents in conjunction with lanthanum salts because of undesirable effects due to interference with the chelating agent.

U.S. Patent 5,625,167 describes a process for the removal of minerals from hair, which includes the use of "synergistic combinations" of chelating agents at a pH range of between 5 to 8. The combination of chelating agents is recommended as no chelating agent used alone adequately removes metal ions from the hair.

U.S. Pat. No. 5,804,172 disclosed a composition for the removal of minerals, which includes the combination of acidifying agent, reducing agent, chelating agent, and gelling agent. The reasoning behind this composition is that, since chelation with EDTA has only marginal efficiency, the inclusion of an acidifying agent and a reducing agent was required to weaken the bonds between the minerals and the hair proteins.

In spite of the documented scientific and patent literature attesting to the absence of an effective hair cleansing treatment for metal contaminants, there are numerous commercial products on the market which claim the ability to demineralize contaminated hair. These claims are usually made, based on the mere inclusion in these products of a chelating agent. It has been discovered that these products are deficient, however, in that the metal salts can redeposit onto the hair.

A metal-cleansing treatment for hair, therefore, is desperately needed. This cleansing treatment should be safe, effective against a wide range of divalent and trivalent metals, and mild enough to lift only metal and soil contaminants from the hair without modifying its color or affecting the integrity and structure of the fiber.

### SUMMARY OF THE INVENTION

The aqueous composition suitable for removing metals, metal ions, metal salts, environmental dirt, and sebum from hair comprises:
(a) an effective amount of an anionic dispersant, and
(b) the remainder being water,
wherein the aqueous composition is at a pH between 2 and 6.0.

The composition can also contain:
(c) an effective amount of a reducing agent, and
(d) an effective amount of a chelating agent.

If the composition is to be used as a demetallizing agent then, in addition to components (a), (b), (c), and (d) above, the composition also contains: (e) an effective amount of a threshold scale inhibitor and (f) an effective amount of a gelling agent.

If the composition is to be used as a clarifying shampoo for the cleansing of hair, in addition to components (a), (b), (c), and (d) above, the composition also contains: (g) an effective amount of a surfactant component selected from the group consisting of anionic surfactants and amphoteric surfactants.

The method for treating hair according to the present invention comprises contacting the hair with any of the compositions above.

### DETAILED DESCRIPTION OF THE INVENTION

This invention provides a composition suitable for removing minerals from hair and preventing them from redepositing onto hair such that the hair, after coming in contact with the composition of this invention will be cleansed and free of excess heavy metal and hard water metal ions. The composition unexpectedly disperses the metal salts in the aqueous environment surrounding the hair after removal and prevents redeposition of the metal salt on another anionic site along the keratin fiber of hair.

It has been unexpectedly found that the combination of (a) an anionic dispersant applied at acidic pH (e.g. 2-6) can effectively remove excess multivalent metal ions, their corresponding salts and other minerals from the surface of hair. This effect can be augmented by the inclusion of (c) a reducing agent, and (d) at least one chelating agent.

If the composition is to be used as a demetallizing agent then, in addition to components above, the composition also contains (e) a threshold scale inhibitor and (f) a gelling agent. If the composition is to be used as a clarifying shampoo for the cleansing of hair, the composition, instead of containing (e) a threshold scale inhibitor and (f) a gelling agent, contains (g) a surfactant component that is selected from the group consisting of anionic surfactants and amphoteric surfactants.

These compositions can provide improved removal of undesirable minerals from hair. As discussed previously, any composition for removing multivalent metal ions from hair can be subject to the multivalent metal ions redepositing onto the hair. This can lead to obvious inadequacies for a product as the desired benefit of excess mineral removal from hair will not be achieved.

The inventor has discovered that through the use of proper pH adjustment to a value at or near the isoelectric point of the keratin hair fiber, the effective charge on the hair becomes zero, or near zero. This decrease in the anionic charge on the hair greatly reduces the driving force that holds the cationic multivalent metal ion onto the hair surface. Through the use of an appropriately strong acid containing anionic dispersant, the multivalent metal ion can be lifted from the hair and removed into the surrounding aqueous environment as a stabilized dispersion. Depending on the nature of a particular multivalent metal ion, the use of a reducing agent to achieve a lower oxidation state of the metal will improve the ability of the dispersant to perform its function. Additionally, if hard water salt crystals are deposited onto the hair, the use of a threshold scale inhibitor will aid the dispersant in performing its function of lifting the crystal from the hair and stabilizing its suspension in the surrounding aqueous environment. The use of a chelant can also, optionally be applied as the chelated metal will be easier for the dispersant to lift from the hair and suspend in the surrounding aqueous environment.

The present invention also provides for compositions that are intended to remove environmental contaminants, sebum as well as minerals from the hair. These compositions will contain the elements outlined above and, for a clarifying shampoo, also contains a sufficient quantity of surface active agent to cleanse the hair of environmental contaminants and sebum.

The inventive composition, when used as a demineralizing treatment will preferably comprise an aqueous solution of pH from 2 to 6, and the effective amount of the anionic dispersant of (a) is about 0.1 to about 10% by weight; the effective amount of the reducing agent of (c) is about 0.05 to about 5% by weight; the effective amount of the chelating agent of (d) is about 0.05 to about 5% by weight; the effective amount of the threshold scale inhibitor of (e) is about 0.05 to about 5% by weight; and the effective amount of the gelling agent of (f) is about 0.05 to about 5% by weight. The compositions are able to remove metallic minerals from the hair protein and stabilize them in the surrounding aqueous environment without redeposition of the metallic minerals onto the hair.

The present invention also provides for a shampoo composition that will both cleanse the hair as well as removing metallic minerals from the hair protein. The clarifying shampoo composition preferably comprises an aqueous solution of pH from 2 to 6, and the effective amount of the anionic dispersant of (a) is about 0.1 to about 10% by weight; the effective amount of the reducing agent of (c) is about 0.05 to about 5% by weight; the effective amount of the chelating agent of (d) is about 0.05 to about 5% by weight; and the effective amount of the surfactant component of (g) is about 1 to about 25% by weight. The compositions are able to remove environmental dirt and sebum as well as metallic minerals from the hair protein and stabilize them in the surrounding aqueous environment without redeposition of the metallic minerals onto the hair until rinsing the composition from the hair is complete.

The aqueous compositions according to the present invention are acidic and are preferably at a pH between about 2 and 6, more preferably at a pH between about 3 and 5. At a pH much below 2, extended exposure to the composition causes skin irritation and can damage hair. At a pH much above 6, the removal of minerals etc. is not very effective.

The compositions according to the present invention preferably contain about 0.1 to about 10% by weight of the anionic dispersant (a), more preferably about 0.1 to about 5, with a % by weight of about 1 to about 3 being most preferred. At amounts much below 0.1 the effect of dispersing the hair contaminants is minimal, whereas at concentrations much above 10% by weight only increase the cost of the composition without providing much additional dispersing.

The compositions according to the present invention preferably contain about 0.05 to about 5% by weight of the reducing agent (c), more preferably about 0.1 to about 2, with a % by weight of about 0.5 to about 1.5 being most preferred. At concentrations much below 0.05 the effect of the reducing agent is minimal, whereas at concentrations much above 5% by weight, hair damage and skin irritation can occur.

The compositions according to the present invention preferably contain about 0.05 to about 5% by weight of the chelating agent (d), more preferably about 0.1 to about 2, with a % by weight of about 0.5 to about 1.5 being most preferred. At concentrations much below 0.05 the effect of the chelating agent is minimal, whereas at concentrations much above 5% by weight, costs increase with little added benefit, while hair damage and skin irritation can occur. At very high concentrations, the chelating agent can pull the metals from the hair that are not surface metals but are part of the hair structure, damaging the hair.

The compositions according to the present invention preferably contain about 0.05 to about 5% by weight of the threshold scale inhibitor (e), more preferably about 0.1 to about 2, with a % by weight of about 0.5 to about 1.5 being most preferred. At concentrations much below 0.05 the effect of the threshold scale inhibitor is minimal, whereas at concentrations much above 5% by weight, only increase the cost with not much added benefit.

The demineralizing compositions according to the present invention preferably contain about 0.05 to about 5% by weight of the gelling agent (f), more preferably about 0.1 to about 2, with a % by weight of about 0.5 to about 1.5 being most preferred. The function of the gelling agent is to thicken the composition and hold it onto the hair. At concentrations much below 0.05 the effect is minimal and the composition will just run off of the hair. Low concentrations of the gelling agent would be fine for shampoos but not for a demineralizing composition that is intended to set on hair for a period of time prior to being rinsed. At concentrations of gelling agent much above 5% by weight, the composition would be so thick that it could not have intimate contact with all of the hair to treat it.

The clarifying shampoo compositions according to the present invention generally can contain about 1 to about 25% by weight of the surfactant component (g), preferably about 3 to about 20, more preferably about 6 to about 15, with a % by weight of about 8 to about 14 being most preferred. At concentrations much below about 1%, the effect of the surfactant component is minimal and the composition would not significantly cleans the hair, whereas at concentrations much above 25% by weight, cost would increase without much added benefit.

The present composition can be made in a dry form, but to be used it needs to be added to water. Thus, the useful form of the present invention an aqueous composition. Water is the majority of the composition and can be as high as about 98% by weight, particularly in the demineralizing composition. Water can be as low as about 60% by weight, particularly in the clarifying shampoo composition since the surfactant component can be present in high concentration, much higher than the other components. The amount of water can thus vary, preferably from about 98 to about 60% by weight, more preferably about 97 to about 80% by weight.

A specific preferred demineralizing composition according to the present invention comprises an aqueous solution of pH of about 3 to about 5, about 1% to about 3% dispersant, about 0.1 to about 1.0% reducing agent, about 0.05% to about 1.0% chelating agent, about 0.05 to about 1.0% threshold scale inhibitor and about 0.1 to about 2.0% gelling agent.

The demineralizing composition can also be supplied as a dry powder, which is subsequently dissolved in water and applied to the hair. A specific preferred dry demineralizing composition according to the present invention comprises about 1% to about 5% dispersant, about 0.5% to about 3% reducing agent, about 0.5% to about 3% chelating agent, about 1% to about 25% gelling agent and about 50% to about 80% citric acid as a pH control agent. The dry powder is fully dissolved in water and applied to the hair when used.

A specific preferred clarifying shampoo composition according to the present invention comprises an aqueous solution of pH of about 3 to about 5, about 1% to about 3% dispersant, about 0.1 to about 1.0% reducing agent, about 0.05% to about 1.0% chelating agent, about 0.05 to about 1.0% threshold scale inhibitor about 5% to about 10% anionic surfactant and about 0.1 to about 5% amphoteric surfactant.

### Dispersant Agent (a)

The demineralizing and clarifying shampoo compositions of the present invention comprise one or more water-soluble anionic dispersants. Effective dispersants will include any anionic material that is capable of lifting multivalent metal salts from the hair and suspending them in the aqueous environment around the hair, preventing redeposition. Specific classes of chemistry that are particularly effective include low molecular weight homopolymers and copolymers of acrylic and/or methacrylic acid, phosphates and polyphosphates and low molecular weight polysaccharides and cellulosics that have been modified to have an anionic charge.

The low molecular weight homopolymers and copolymers of acrylic and/or methacrylic acid are obtained from conventional radical polymerization procedures in the presence of polymerization regulators, such isopropanol, bisulfite or mercaptan compounds and in the presence of polymerization catalysts such as peroxides and persalts, e.g., oxygenated water persulfate, sodium hypophosphite, hypophosphorous acid. The following monomers and/or co-monomers may be used: acrylic acid, methacrylic acid, 2-acrylamido-2-propane sulfonic acid (AMPSA), 2-methacrylamido-2-propane sulfonic acid, itaconic acid, crotonic acid, fumaric acid, maleic anhydride acid, or, alternatively, isocrotonic acid, aconitic acid (cis or trans), mesaconic acid, sinapinic acid, undecylenic acid, angelic acid, canellic acid, or hydroxyacrylic acid, sulfonated styrene, vinyl sulfonate, , 3-allyloxy-2-hydroxy propyl sulfonic acid, vinyl phosphonic acid, and sulphenoxy methallyl ether, existing either as free acids or partially neutralized salts, acrolein, acrylamide, acrylonitrile, the esters of acrylic and methacrylic acids, and, in particular, dimethylaminoethyl methacrylate, vinylpyrrolidone, vinylcaprolactam, ethylene, propylene, isobutylene, diisobutylene, vinyl acetate, styrene, α-methyl styrene, methyl vinyl ketone, and hydroxy propyl acrylate.

The phosphates and polyphosphates that can be used include, but are not limited to polyphosphates and sodium hexametaphosphate.

The low molecular weight polysaccharides and cellulosics that have been modified to have an anionic charge that can be used include, but are not limited to carboxymethyl inulin, dicarboxy inulin, carboxymethyl cellulose, sulfonated lignin, carboxylated lignin, oxidized lignin and salts thereof.

Specific preferred examples of the anionic dispersant according to the present invention include a polymer comprised of about 60 weight % acrylic acid and 40 weight % 2-methacrylamido-2-propane sulfonic acid available from Calgon Corporation as pHREEGuard 4500, carboxymethyl inulin such as the CMxxPP line of carboxymethylinulin materials from COSUN, The Netherlands, dicarboxy inulin such as the DCxxPP line of dicarboxyinulin materials from COSUN (xx is an integer from 0 to 100) and/or sodium hexametaphosphate such as Calgon R available from Calgon Corporation.

### Reducing Agent (c)

A reducing agent is a material that readily gives up electron(s) to another element or compound, causing it to be reduced. This reducing agent causes a metal cation of a higher charge to accept an electron, and consequently, assume a lower cationic charge. In the case of iron, the ferrous form is much more soluble than the ferric. It is easily chelatable, and /or dispersible. Therefore, by adding sufficient amounts of the reducing agents according to the process of the present invention, the metals are reduced to the lower oxidation state, such as iron (III) to iron (II), allowing the easier formation of chelates.

Reducing agents that are useful in the composition and process of the present invention can be organic, such as ascorbic acid and erythorbic acid and can be inorganic reducing agents that are selected from the group consisting of sulfites (including hydrogen sulfites), thiosulfates, persulfates, and peridates, including the ammonium, potassium, or sodium salts thereof. Sulfite inorganic reducing agents are preferred, with sulfite inorganic reducing agents selected from the group consisting of bisulfites and metasulfites being more preferred. The more preferred are the salts of hydrogen sulfites, with sodium bisulfite and sodium hydrogen sulfite being most preferred.

A reducing agent will be included in the inventive composition to transfer electrons to the oxidized iron, copper, lead, chromium and tin and other oxidized metals to reduce their oxidation state and thereby lower the strength of the cysteine-metal bond. For example, the reduction of iron from Fe⁺³ to Fe⁺² reduces the strength of the cysteine-iron bond thereby increasing the ease of removal by the dispersant. Hypophosphourous acid, ascorbic acid and erythorbic acid are preferred reducing agents.

### Chelating Agents (d)

Chelating agents are organic compounds in which atoms form more that one coordinate bond with metals in solution. The presence of a chelant according to the present invention allows some of the reduced metals (such as iron) to be held in solution further preventing re-deposition onto the surface of the hair. The Chelants in the process of the present invention function by "locking" the metal ion into a soluble organic ring structure. The chelant can also solubilize unreacted metals as well as the reduced metals.

Chelating agents of the present invention are preferably selected from the group consisting of phosphonates and polycarboxylic acids (including polyamino carboxylic acids), and are more preferably selected from the group consisting of ethylene diamene tetraacetic acid (EDTA), dicarboxy inulin, maleic acid, oxalic acid, citric acid, and their salts, with the more preferred chelants being salts of EDTA and of citrates with the most preferred being salts of EDTA and dicarboxy inulin for most effective use of a chelant in the pH range used in the method according to the present invention. Due to cost and efficacy, dicarboxy inulin and the sodium salt of ETDA are most preferred.

A strong chelating agent can be added to sequester the metal ions and facilitate removal by the dispersant. The acidic nature of the inventive compositions, in combination with the reducing agent reduces the metal cysteine bond strength to the point that the mixture of chelators or chelator and dispersants or dispersant may be used effectively.

The most preferred chelating agents include EDTA and dicarboxy inulin such as the DCxxPP line of dicarboxyinulin materials from COSUN (part of SENSUS of the Netherlands).

### Threshold Scale Inhibitor (e)

In the event that hard water salt crystals are deposited on the hair, the use of a threshold scale inhibitor can be employed to aid the dispersant in removing it from the surface of the hair.

Almost 65 years ago, it was discovered that certain inorganic polyphosphates would prevent precipitation of hard water salts when added in amounts far less than the concentrations needed for sequestering or chelating. By polyphosphates, we mean phosphates having a molar ratio of metal oxide:P₂O₅ between 1:1 and 2:1.

Optionally, any water-soluble phosphate may be used to augment the use of a dispersant. Examples include orthophosphates; condensed phosphates, such as sodium hexametaphosphate; phosphate esters; organophosphate esters, such as the lower alkyl mono-, di- and trialkyl phosphates. The alkyl group is selected from C₁ to C₄ and may be branched or unbranched. The alkyl group may be substituted with hydroxy, amino, halide, sulfate or sulfonate, alone or in combination; and molecularly dehydrated phosphates.

Water-soluble phosphonates have also been used over the years to solubilize hard water deposits. Water-soluble phosphonates which may be included are 2-phosphono-1,2,4-tricarboxybutane, amino tri(methylene phosphonic acid), hydroxyethylidene diphosphonic acid, phosphonosuccinic acid, benzene phosphonic acid, 2-aminoethyl phosphonic acid, polyamino phosphonates and the like. Additional phosphonates are identified in U.S. Pat. No. 3,837,803, which is hereby incorporated by reference. The preferred phosphonates are 2-phosphono-1,2,4-tricarboxybutane, amino tri(methylene phosphonic acid) and hydroxyethylidene diphosphonic acid.

### pH

The pH of the compositions of the present invention is acidic, preferable from 2 to 6, more preferably from 3 to 5. The acidic pH decreases the anionic charge on the hair protein as well as increasing the solubility of many of the hard water salts that deposit onto the hair.

### Gelling Agent (f)

A gelling agent is included in the inventive composition to retain the composition in intimate contact with the hair shaft. The gelatinous consistency of the inventive composition is necessary to retain the composition on the hair shaft and allows the hair stylist to force the compositions into the hair shaft by squeezing with application of firm pressure. Suitable examples of gelling agents include: xanthan gum, carrageenan, cellulose, cellulose gum, methyl cellulose, carbomer, carboxymethyl cellulose, hydroxy ethyl cellulose, guar, hydroxy ethyl ethyl cellulose, hydroxypropyl cellulose, chitosan, hydroxypropyl chitosan, hydroxypropyl starch, starch, hydroxypropyl guar, cyclodextrin, hydroxypropyl cyclodextrin, hydroxypropyl methyl cellulose, gelatin, hydroxypropyl gelatin, protein, hydroxypropyl protein, polyacrylamide, poly(acrylic acid), sodium polyacrylate, poly(acrylamidomethylpropane sulfonic acid), poly(acrylamidomethylpropane sulfonic acid) sodium salt, and acrylamide copolymers. A preferred gelling agent is xanthan gum.

### Surfactant Component (g)

### Anionic Surfactant

The shampoo compositions of the present invention preferably contains an anionic surfactant as at least part of component (g), which can comprise one or more anionic detersive surfactants which are anionic at the pH of the shampoo, to provide cleaning performance to the composition.

The anionic surfactant of component (a) can be the only surfactant and will generally be present at a level from about 1% to about 25% by weight.

Anionic detersive surfactants useful herein include those that are disclosed in U.S. Patent No. 5,573,709, the disclosure of which is incorporated herein by reference in its entirety. Examples include alkyl and alkyl ether sulfates. Specific examples of alkyl ether sulfates which may be used In the present invention are sodium and ammonium salts of lauryl sulfate, lauryl ether sulfate, coconut alkyl triethylene glycol ether sulfate; tallow alkyl triethylene glycol ether sulfate, and tallow alkyl hexaoxyethylene sulfate. Highly preferred alkyl ether sulfates are those comprising a mixture of individual compounds, said mixture having an average alkyl chain length of from about 12 to about 16 carbon atoms and an average degree of ethoxylation of from about 1 to about 6 moles of ethylene oxide.

Another suitable class of anionic detersive surfactants is the alkyl sulfuric acid salts. Important examples are the salts of an organic sulfuric acid reaction product of a hydrocarbon of the methane series, including iso-, neo-, ineso-, and n-paraffins, having about 8 to about 24 carbon atoms, preferably about 12 to about 18 carbon atoms and a sulfonating agent, e.g., SO₃, H₂SO₄, oleum, obtained according to known sulfonation methods, including bleaching and hydrolysis. Preferred are alkali metal and ammonium sulfated C₁₂₋₃₈ n-paraffins.

Additional examples of synthetic anionic detersive surfactants which come within the terms of the present invention are the olefin sulfonates, the beta-alkyloxy alkane sulfonates, and the reaction products of fatty acids esterified with isethionic acid and neutralized with sodium hydroxide, as well as succinamates. Specific examples of succinamates include disodium N-octadecyl sulfofosuccinanrate; tetrasodium N-(1,2-dicarboxyethyl) - N-octadecylsulfosuccinamate ; diamyl eater of sodium sulfosuccinic acid; dihexyl ester of sodium sulfosuccinic acid; dioctyl esters of sodium sulfosuccinic acid.

Preferred anionic detersive surfactants for use in the present shampoo compositions include ammonium lauryl sulfate, ammonium laureth sulfate, trlethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, trlethanolamine 1 lauryl sulfate fate, triethanolamine lauryl sulfate, monoethanolamine cocoyl sulfate, monoethanolamine lauryl sulfate, sodium tridecyl benzene sulfonate, and sodium dodecyl benzene sulfonate.

### Amphoteric Surfactant

The hair conditioning shampoo composition of the present invention preferably contains an amphoteric detersive surfactants, more preferably in combination with the anionic surfactant. The amount of this surfactant is preferably no more than about 10 weight %. Examples of amphoteric detersive surfactants which can be used in the compositions of the present invention are those which are broadly described as derivatives of aliphatic secondary and tertiary amines in which the aliphatic substituent contains from about 8 to 18 carbon atoms and one contains an anionic water solubilizing group e.g., carboxy, sulfonate, sulfate, phosphate, or phosphonate. Examples of compounds falling within this definition are sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Patent 2,658,072, N-higher alkyl aspartic acids such as those produced according to the teaching of U.S. Patent 2,438,091, and the products sold under the trade name "MIRANOL"™ as described in U.S. Patent 2,528,378.

Included as a subset of amphoteric surfactants, are zwitterionics such as betaines and can also useful in the present invention. Examples of betaines useful herein include the high alkyl betaines, such as coco dimethyl carboxymethyl betaine, cocoamidopropyl betaine, cocobetaine, lauryl amidopropyl betaine, oleyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, and lauryl bis-(2-hydroxypropyl)alpha-carboxyethyl betaine. The sulfobetaines may be represented by coco dimethyl sulfopropyl betaine, stearyl dimethyl sulfopropyl betaine, lauryl dimethyl sulfoethyl betaine, lauryl bis-(2-hydroxyethyl) sulfopropyl betaine and the like ; amidobetaines and amidosulfobetaines, wherein the RCONH(CH₂)₃ radical is attached to the nitrogen atom of the betaine are also useful in this invention.

Preferred shampoos of the present invention contain combinations of anionic surfactants with zwitterionic surfactants and/or amphoteric surfactants. Especially preferred shampoos contain a minor amount up to about 16% by weight, preferably from about 3% to about 16% of alkyl sulfates; a minor amount up to 16% by weight, preferably from about 3% to about 12% of ethoxylated alkyl sulfates; and a minor amount up to 10% by weight, preferably from about 1% to about 8% of optional detersive surfactants selected from the amphoteric and zwitterionic detersive surfactants, with at least 5% of either alkyl sulfate, ethoxylated alkyl sulfate, or a mixture thereof, and a total surfactant level of from about 6% to about 20%.

### EXAMPLES

### Example 1

The following ingredients were added to water under constant agitation.

### Demineralizing Gel

| Component & Component letter | weight | wt. % |
|---|---|---|
| Xanthan Gum (f) | 2.50g | 1.0 |
| 50% Aqueous Hypophosphourous Acid (c) | 1.25g | 0.25 |
| Disodium EDTA (d) | 0.25g | 0.1 |
| 60% Aqueous Hydroxyethylidene Diphosphonic Acid (e) | 0.25g | 0.06 |
| 60% Aqueous Pyrophosphate(e) | 0.25g | 0.06 |
| 45% Aqueous AA/AMPSA copolymer* (a) | 5.00g | 0.9 |
| 10% Aqueous sodium hydroxide (pH adjustment) | 5.50g | 0.22 |
| Deionized Water (b) | 240.5g | 97.41 |
| Final pH = 4.1 | | |

| | | |
|---|---|---|
| *AA - Acrylic Acid *AMPSA - 2-Acrylamido-2-methylpropanesulfonic acid | | |

### Example 2

Three hair swatches from the same lot of hair were prepared as outlined below.

### Demineralizing Performance

Sample 1 (control) - Untreated bleached brown hair from DeMeo Brothers
Sample 2 (control) - Bleached brown hair soaked in synthetic hard water for 16 hours
Sample 3 (inventive) - Bleached brown hair soaked in synthetic hard water for 16 hours, blown dry with a standard hair dryer and soaked in the treatment of Example 1 for 60 minutes at 120°F and rinsed for 90 seconds with deionized water.

### Synthetic Hard Water

| | |
|---|---|
| Tap Water | 1,000g |
| CaCl₂·H₂O | 0.6g |
| MgSO₄ | 0.2g |
| Na₂SiO₃ | 0.2g |

The amount of Calcium and Magnesium on the hair tresses was measured by atomic adsorption spectroscopy after digestion of the hair samples. The results are an average of three experiments at each condition.

| | Calcium (ppm) | Magnesium (ppm) |
|---|---|---|
| Sample 1 (control) | 5,820 | 763 |
| Sample 2 (control) | 12,801 | 1,531 |
| Sample 3 (inventive) | 4,218 | 399 |

The data demonstrate the ability of the composition of Example 1 to remove hard water deposits from the hair.

### Example 3

The following components are dry blended to form a powder. This composition can then be dissolved into water at the time of its use.

### Demineralizing Powder

| Component & Component Letter | Concentration |
|---|---|
| Xanthan Gum (f) | 20% |
| Sodium Hypophosphite (c) | 2% |
| Sodium Hexametaphosphate (a) | 10% |
| Disodium EDTA (d) | 2% |
| Citric Acid (pH adjustment) | 76% |

### Example 4

The following ingredients were added to water under constant agitation as in Example 1.

### Natural Derived Demineralizing Gel

| Component & Component letter | Weight | wt. % |
|---|---|---|
| Xanthan Gum (f) | .50g | 1.0 |
| Carboxymethyl Inulin¹ (a) | 5.00g | 2.0 |
| Dicarboxy Inulin² (d) | 0.50g | 0.2 |
| Ascorbic Acid (c) | 0.50g | 0.2 |
| Citric Acid (pH adjustment) | 0.50g | 0.2 |
| Deionized Water (b) | 241.0g | 96.4 |
| Final pH = 5.3 | | |

| | | |
|---|---|---|
| ¹ Carboxymethylated Inulin (degree of substitution = 2.2) derived from chicory used as a dispersant | | |
| ² Oxidized Inulin (20% dicarboxylated) derived from chicory used as a chelating agent | | |

### Example 5

Three hair tresses from the same lot of bleached brown hair were prepared as in Example 2:
Sample 4 (control) - Bleached Brown Hair
Sample 5 (control) - Bleached Brown Hair soaked in synthetic hard water
Sample 6 (inventive) - Bleached Brown Hair soaked in synthetic hard water and treated with the formula of this example at 84 - 95°F for 28 minutes and rinsed for 30 seconds with deionized water.

The amount of Calcium and Magnesium on the hair tresses was measured by atomic adsorption spectroscopy after digestion of the hair samples.

| | Calcium (ppm) | Magnesium (ppm) |
|---|---|---|
| Sample 4 | 5,610 | 705 |
| Sample 5 | 11,559 | 1,844 |
| Sample 6 | 2,267 | 402 |

The data demonstrate the ability of the composition of Example 4 to remove hard water deposits from the hair.

## Claims

1. An aqueous composition suitable for removing metals, metal ions, metal salts, environmental dirt, and sebum from hair comprising:
a. effective amount of an anionic dispersant, and
b. the remainder being water,
wherein the aqueous composition is at a pH between 2 and 6.

2. The composition according to claim 1 further comprising:
c. an effective amount of a reducing agent, and
d. an effective amount of a chelating agent.

3. The composition according to claim 2 further comprising:
e. an effective amount of a threshold scale inhibitor and
f. an effective amount of a gelling agent.

4. The composition according to claim 2 further comprising:
g. an effective amount of a surfactant component selected from the group consisting of anionic surfactants and amphoteric surfactants.

5. The composition according to claim 3 wherein the composition is a demetalizing composition and the effective amount of the anionic dispersant of (a) is 0.1 to 10% by weight; the effective amount of the reducing agent of (c) is 0.05 to 5% by weight; the effective amount of the chelating agent of (d) is 0.05 to 5% by weight; the effective amount of the threshold scale inhibitor of (e) is 0.05 to 5% by weight; and the effective amount of the gelling agent of (f) is 0.05 to 5% by weight.

6. The composition according to claim 4 wherein the composition is a clarifying shampoo and the effective amount of the anionic dispersant of (a) is 0.1 to 10% by weight; the effective amount of the reducing agent of (c) is 0.05 to 5% by weight; the effective amount of the chelating agent of (d) is 0.05 to 5% by weight; and the effective amount of the surfactant component of (g) is 1 to 25% by weight.

7. The composition of claim 1 wherein the anionic dispersant of (a) is a polymer made from at least one monomer selected from the group consisting of the following; acrylic acid, methacrylic acid, 2-acrylamido-2-propane sulfonic acid (AMPSA), 2-methacrylamido-2-propane sulfonic acid, itaconic acid, crotaonic acid, fumaric acid, maleic anhydride acid, isocrotonic acid, aconitic acid (cis or trans), mesaconic acid, sinapinic acid, undecylenic acid angelic acid, canellic acid, or hydroxyacrylic acid, sulfonated styrene, vinyl sulfonate, 3-allyloxy-2-hydroxy propyl sulfonic acid, vinyl phosphonic acid, and sulphenoxy methallyl ether, existing either as free acids or partially neutralized salts, acrolein, acrylamide, acrylonitrile, the esters of acrylic and methacrylic acids, dimethlaminoethyl methacrylate, vinylpyrrolidone, vinylcaprolactam, ethylene, propylene, isobutylene, diisbutylene, vinyl acetate, styrene, α-methyl styrene, methyl vinyl ketone, and hydroxy propyl acrylate.

8. The composition of claim 1 where the anionic dispersant of (a) is selected from the list of carboxymethyl inulin, dicarboxy inulin, carboxymethyl cellulose, sulfonated lignin, carboxylated lignin, oxidized lignin, polyaspartic acid, and salts thereof.

9. The composition of claim 1 where the anionic dispersant of (a) is sodium hexametaphosphate.

10. The composition according to claim 2 wherein reducing agent of (c) is selected from the group of inorganic reducing agents consisting of sulfites, thiosulfites, persulfates, peridates, hypophosphorous acid, and hypophosphite salts.

11. The composition of claim 2 where the chelating agent of (d) is selected from the group consisting of dicarboxy inulin and ethylene diamen tetraacetic acid (EDTA).

12. The composition of claim 3 where the threshold scale inhibitor (e) is selected from the group consisting of 2-phosphono-1,2,4-tricarboxybutane, amino tri(methylene phosphonic acid) and hydroxyethylidene diphosphonic acid.

13. The composition of claim 3 where the gelling agent of (f) is selected from the group consisting of xanthan gum, carrageenan, cellulose, cellulose gum, methyl cellulose, carbomer, carboxymethyl cellulose, hydroxy ethyl cellulose, guar, hydroxy ethyl cellulose, hydroxypropyl cellulose, chitosan, hydroxypropyl chitosan, hydroxypropyl starch starch, hydroxypropyl guar, cyclodextrin, hydroxypropyl cyclodextrin, hydroxypropyl methyl cellulose, gelatin, hydroxypropyl gelatin, protein, hydroxypropryl protein, polyacrylamide, poly(acrylic acid), sodium polyacrylate, poly(acrylamidomethylpropane sulfonic acid), poly(acrylamidomethylpropane sulfonic acid) sodium salt, and acrylamide copolymers.

14. The composition of claim 4 wherein the surfactant component of (g) is an anionic surfactant selected from the group consisting of one or more of ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate potassium lauryl sulfate, potassium laureth sulfate, sodium lauryl sarcosinate, sodium lauroyl sarcosinate, lauryl sarcosine, cocoyl sarcosine, ammonium cocoyl sulfate ammonium lauroyl sulfate, sodium cocoyl sulfate, sodium lauroyl sulfate, potassium cocoyl sulfate, potassium lauryl sulfate, triethanolamine 1 lauryl sulfate fate, triethanolamine lauryl sulfate, monoethanolamine cocoyl sulfate, monoethanolamine lauryl sulfate, sodium tridecyl benzene sulfonate, and sodium dodecyl benzene sulfonate.

15. The composition of claim 14 where from 0.1 to 10% of the composition consists of a second surfactant selected from amphoteric or betaine surfactant selected from the group consisting of one or more of coco dimethyl carboxymethyl betaine, cocoamidopropyl betaine, cocobetaine, lauryl amidopropyl betaine, oleyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-2(2-hydroxypropyl) carboxymethyl betaine, oleyl dimethyl gamma-carboxypropyl betaine, and lauryl bis-(2-hydroxypropryl)alpha-carboxyethyl betaine, coco dimethyl sulfopropyl betaine, stearyl dimethyl sulfopropyl betaine, lauryl dimethyl sulfoethyl betaine, lauryl bis-(2-hydroxyethyl) sulfopropryl betaine, sodium 3-dodecyl-aminopropionate, sodium 3-dodecylaminopropane sulfonate and sodium lauryl sarcosinate.

16. A process for demineralizing hair comprising contacting hair for a time with an aqueous demineralizing composition comprising:
a. 0.1 to 10% by weight of an anionic dispersant,
b. water,
c. 0.05 to 5% by weight of a reducing agent,
d. 0.05 to 5% by weight of a chelating agent,
e. 0.05 to 5% by weight of a threshold scale inhibitor, and
f. 0.05 to 5% by weight of a gelling agent; followed by rinsing the hair, wherein the aqueous composition is at a pH between 2 and 6.

17. The process according to claim 16 wherein the demetalizing composition contains (a) 0.1 to 5% by weight of an anionic dispersant, (c) 0.1 to 2% by weight of the reducing agent, (d) 0.1 to 2% by weight of the chelating agent, (e) 0.1 to 2% by weight of the threshold scale inhibitor, and 0.1 to 2% by weight of the gelling agent, with the remainder being water (b).

18. The process according to claim 17 wherein the demetalizing composition contains (a) 1 to 3% by weight of the anionic dispersant, (c) 0.5 to 1.5% by weight of the reducing agent, (d) 0.5 to 1.5% by weight of the chelating agent, (e) 0.5 to 1.5% by weight of the threshold scale inhibitor, and 0.5 to 1.5% by weight of the gelling agent, with the remainder being water (b).

19. A process for cleansing hair comprising contacting hair for a time with an aqueous clarifying shampoo composition of claim 16 followed by rinsing the hair, wherein the aqueous composition is at a pH between 2 and 6.

20. The process according to claim 19 wherein the clarifying shampoo composition contains (a) 1 to 3% by weight of the anionic dispersant, (c) 0.5 to 1.5% by weight of the reducing agent, (d) 0.5 to 1.5% by weight of the chelating agent, (g) 6 to 15% by weight of the surfactant component, with the remainder being water (b).
